(19) Europäisches Patentamt
European Patent Office
Office européen des brevets

(11) Publication number : **0 546 661 A1**

# (12) EUROPEAN PATENT APPLICATION

(21) Application number : **92309244.9**

(51) Int. Cl.$^5$ : **C07D 239/62,** C07D 239/60

(22) Date of filing : **09.10.92**

A request for correction of the description has been filed pursuant to Rule 88 EPC. A decision on the request will be taken during the proceedings before the Examining Division (Guidelines for Examination in the EPO, A-V, 2.2).

(30) Priority : **09.10.91 JP 290538/91**
**14.09.92 JP 271161/92**

(43) Date of publication of application :
**16.06.93 Bulletin 93/24**

(84) Designated Contracting States :
**BE DE DK ES FR GB IT LU NL PT SE**

(71) Applicant : **NIPPON MINING COMPANY LIMITED**
**10-1, Toranamon 2-chome**
**Minato-ku Tokyo (JP)**

(72) Inventor : **Hirota, Kosaku**
**22-5, Mitahora Higashi 3-chome**
**Gifu-shi, Gifu (JP)**
Inventor : **Fukazawa, Tominaga**
**Nippon Mining Co. Ltd, 17-35, Niizominami, 3-chome**
**Toda-shi, Saitama (JP)**
Inventor : **Isobe, Yoshiaki**
**Nippon Mining Co. Ltd, 17-35, Niizominami, 3-chome**
**Toda-shi, Saitama (JP)**
Inventor : **Morita, Hiroyuki**
**Nippon Mining Co. Ltd, 17-35, Niizominami, 3-chome**
**Toda-shi, Saitama (JP)**

(74) Representative : **Davies, Jonathan Mark**
**Reddie & Grose 16 Theobalds Road**
**London WC1X 8PL (GB)**

## (54) Pyrimidine derivatives, method for preparation thereof and pharmaceutical composition having said derivatives as active elements.

(57) The invention provides i)
pyrimidine derivatives of the following formula (I), their pharmaceutically acceptable salt and method for preparation thereof.

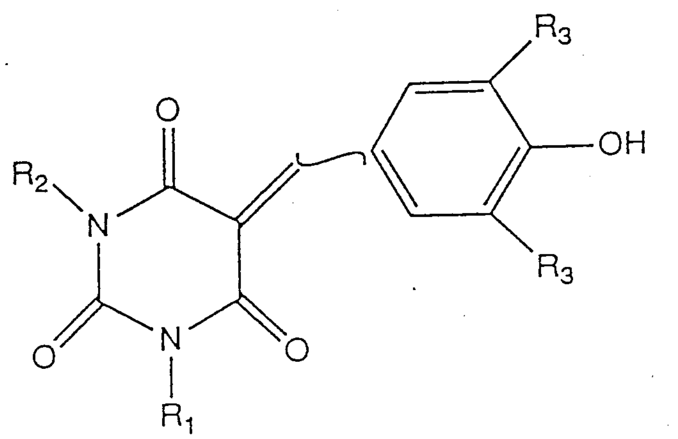

(I)

($R_1$ and $R_2$ represent independently hydrogen atom, linear or branched alkyl group, substituted or non-substituted phenyl or cyclohexyl group ; and $R_3$ represents linear or branched lower alkyl or alkoxy group ; 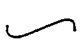 represents both stereo isomers.) ; and ii)
Pharmaceutical compositions having the pyrimidine derivatives of formula (I) or pharmaceutically acceptable salts thereof, particularly anti-inflammatory agents, agents for treating respiratory tract disorders or agents for treating cerebrovascular diseases.

EP 0 546 661 A1

## Field of the Invention

The present invention relates to novel pyrimidine derivatives, method for preparation thereof and pharmaceutical composition having said derivatives as active elements, particularly for the treatment of inflamation like rheumatism, respiratory tract disorders or cerebrovascular diseases.

## Description of the Prior Art

Conventionally, non-steroid type anti-inflammatory agents such as indomethacin and sodium diclofenac were used for the treatment of diseases like arthritis and rheumatism. However, these drugs have side-effect which is frequent occurrence of ulcers in the digestive system. The cyclooxygenase inhibition being the basis of such side-effect as well as of the efficacy of the drug, such side-effect is inevitable disadvantage for the drugs of this type.

Using aforesaid conventional anti-inflammatory agents for the treatment of diseases like rheumatism where a long term administration is required, ulcers in the digestive system are often observed as side-effects. This is the reason why an "anti-rheumatism agent without causing ulcers" was desired.

Recently, active oxygen and leukotriens as the metabolite generated from arachidonic acid by the action of 5-lipoxygenase, are known to have significant relationship with various inflammatory disorders such as rheumatism [J. Med. Chem., 30, 1995-1998 (1987), Science, 220, 568-575 (1983), Am. J. pathol., 107, 397-418 (1982)]. Such information lead us to think that those agents having a suppressive action to these factors relating to the inflammation and, at the same time, not having an action to cause ulcers in the digestive system can be excellent anti-inflammatory agents.

On the other hand, for the treatment of respiratory tract disorders such as bronchitis or asthma, drugs like anti-histamine agents, theophylline, β2 stimulant or steroids are generally used. These compounds other than steroids are effective against immediate asthmatic response (IAR), however, they are not sufficiently effective against late asthmatic response (LAR). Steroids are effective against LAR but are difficult to use in long term because of its various side-effects. Thus, development of non-steroid type drugs which are effective against both IAR and LAR are desired.

Recently, it has been reported that active oxygen species is deeply associated with the respiratory tract disorders. That is, active oxygen species is generated during the IAR and cause edema in the bronchi or cause tearing off of the bronchial mucosa to lead the disease to LAR (Medical Immunology, 15, 61-71 (1988)). These information lead us to think agents having activity to scavenge the active oxygen ,species and, at the same time, to dilate the bronchia will be an excellent drug for treating IAR and LAR.

Moreover, it has turned out that the active oxygen species is also associated with cerebrovascular diseases such as cerebral thrombosis [Drug Develop. Res., 6, 339-344 (1985), Eur. J. Pharmacology, 197, 75-82 (1991)]. That is, the active oxygen species, generated upon the re-perfusion after the treatment with thrombolytic agents, cause the formation of edema and damage the brain to lead to the aftereffects. To prevent such aftereffects, administering the active oxygen species scavengers with the thrombolytic agents is considered to be effective.

## Summary of the Invention

The present invention was made under the aforesaid background. That is, the object of the present invention is to provide the compounds which suppress the factors (such as leukotriens or active oxygen species) associated with inflammation and to provide the method for preparation thereof. Furthermore, the object of the present invention is to provide the pharmaceutical composition having such compounds as active elements, particularly anti-inflammatory agents, agents to treat respiratory tract disorders or cerebrovascular diseases.

The present inventors have actively studied abovementioned subject and found out the compounds described in Formula (I) and pharmaceutically acceptable salt thereof have excellent anti-inflammatory activity, and furthermore, have efficacy against respiratory tract disorders such as asthma or cerebrovascular diseases such as cerebral infarction.

The present invention is related to the pyrimidine derivatives described in the formula (I) and their pharmaceutically acceptable salt.

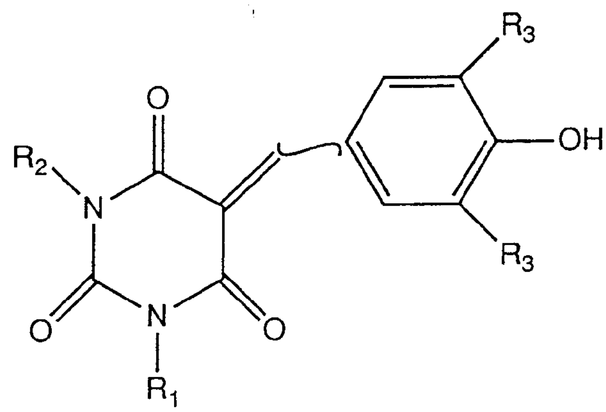

(I)

($R_1$ and $R_2$ represent independently hydrogen atom, linear or branched alkyl group, substituted or non-substituted phenyl or cyclohexyl group, and $R_3$ reprsents linear or branched lower alkyl or alkoxy group.  represents to contain both stereo isomers.)

Furthermore the present invention relates to the method for preparation of the compound described in the formula (I) by reacting the pyrimidine derivatives described in the following formula (II) and hydroxybenzaldehyde derivatives described in the formula (III).

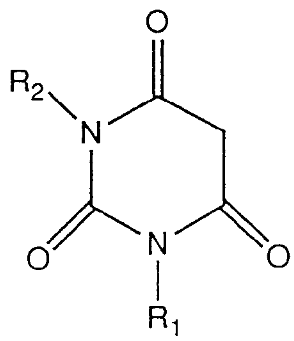

(II)

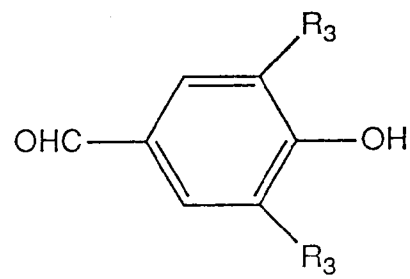

(III)

($R_1$, $R_2$ and $R_3$ represent the same thing as described in the legend of formula (I).)

Furthermore, the present invention relates to pharmaceutical compositions having the pyrimidine derivatives described in the formula (I) or pharmaceutical acceptable salt thereof.

**Description of Preferred Embodiment**

A further detailed description of the formula (I) is the followings. The lower alkyl group means alkyl groups with 1 to 5 carbons, for example, methyl, ethyl, propyl, n-butyl, sec-butyl, tert-butyl and pentyl group.

Substituted phenyl group means benzene ring having its one or multiple hydrogen atoms substituted with lower alkyl group, hydroxyl group and/or halogen atom. The lower alkyl group represents the same with the aforesaid lower alkyl group. Halogen represents fluoride, chloride or bromide.

Examples of the lower alkoxy groups are methoxy, ethoxy, n-propoxy, iso-propoxy n-butoxy, sec-butoxy, tert-butoxy, pentyloxy group, etc.

Examples of the pyrimidine derivatives described in the formula (I) are, 1-methyl-3-phenyl-5-(3,5-di-tert-butyl-4-hydroxybenzylidene)-pyrimidine-2,4,6-trione, 1,3-diethyl-5-(3,5-di-tert-butyl-4-hydroxybenzylidene)-pyrimidine-2,4,6-trione, 1,3-dicyclohexyl-5-(3,5-di-tert-butyl-4-hydroxybenzylidene)-pyrimidine-2,4,6-trione,1-butyl-5-(3,5-di-tert-butyl-4-hydroxybenzylidene)-pyrimidine-2,4,6-trione, 1-cyclohexyl-5-(3,5 -di-tert-butyl-4-hydroxybenzylidene)-pyrimidine-2,4,6-trione, 1-phenyl-5-(3,5-di-tert-butyl-4-hydroxybenzylidene)-pyrimidine-2,4,6-trione, 1,3-ditolyl-5-(3,5-di-tert-butyl-4-hydroxybenzylidene)-pyrimidine-2,4,6-trione, 1-methyl-3-phenyl-5-(3,5-dimethyl-4-hydroxybenzylidene)-pyrimidine-2,4,6-trione, 1-methyl-3-phenyl-5-(3,5-dimethoxy-4-hydroxybenzylidene)-pyrimidine-2,4,6-trione, 1-methyl-3-(4-fluorophenyl)-5-(3,5-di-tert-butyl-4-hydroxybenzylidene)-pyrimidine-2,4,6-trione, 1-methyl-3-(4-chlorophenyl)-5-(3,5-dimethyl-4-hydroxybenzylidene)-pyrimidine-2,4,6-trione, etc.

The formula (I) contains E form and Z form stereo isomers described in the formula (I)-1 and (I)-2 below.

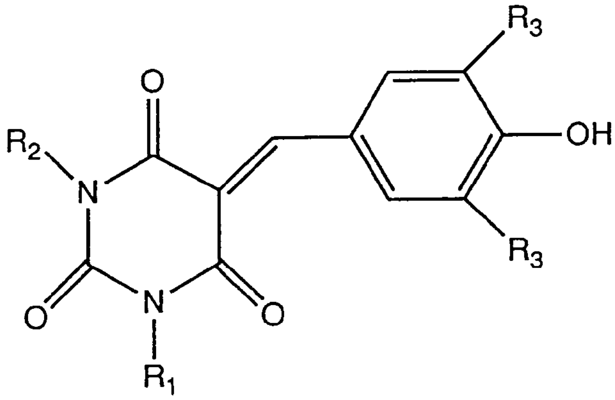

(I)-1

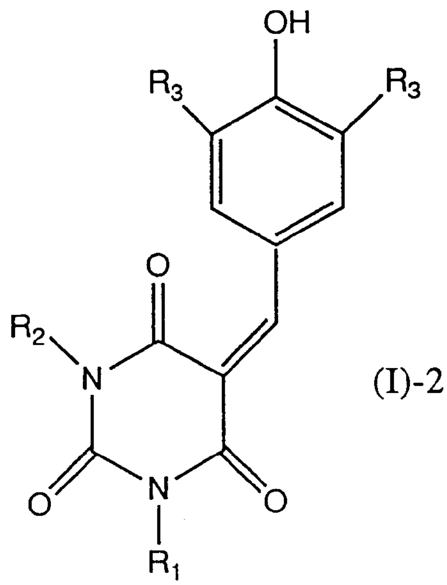

(I)-2

The compounds of the present invention described in the formula (I) contain each of the stereo isomers, and mixture of them in appropriate ratio.

For example, the pyrimidine derivatives described in the formula (I) may be synthesized by reacting the pyrimidine derivatives described in the formula (II) below with the hydroxybenzaldehyde derivatives described in the following formula (III).

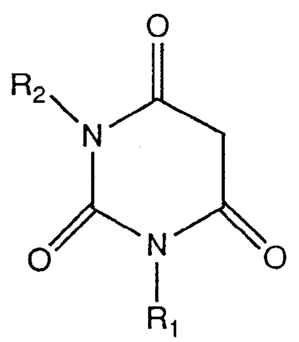

(II)

($R_1$ and $R_2$ represent the same as described in the legend of formula (I))

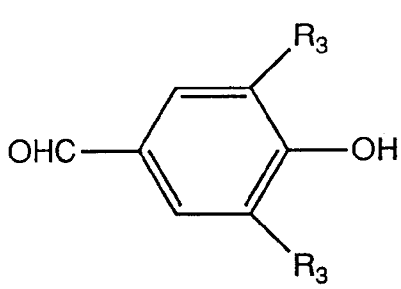

(III)

($R_3$ represents the same as described in the legend of formula (I))

For example, the reactions of the compounds described in the formula (II) and those in the formula (III) may be conducted in appropriate alcoholic solvent such as methanol, ethanol or propanol, generally under the room temperature or under the refluxing temperature of the reaction mixtures.

The compounds in the formula (II) are known and may be prepared by known synthetic method such as reaction of N, N'-substituted urea and diethylmalonate [Chem. Ber., 65, 123-126 (1932)].

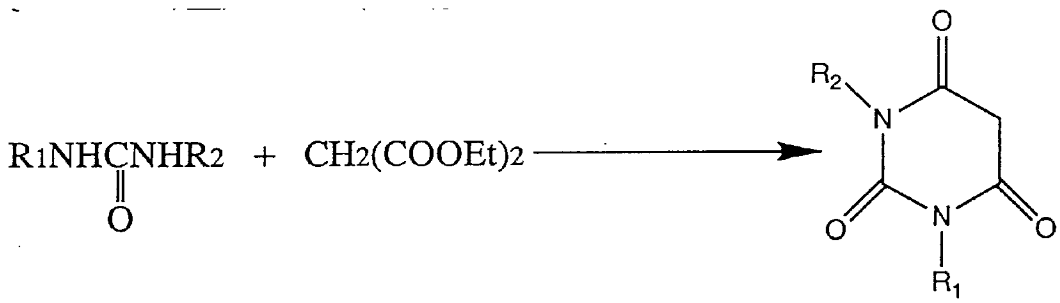

(II)

($R_1$ and $R_2$ represent the same as described in the legend of formula (I))

The compounds described by the formula (III) are commercially available.

Examples of the pharmaceutically acceptable salt are hydrochloride and sulfate, etc.

The compounds described by the formula (I), generated by abovementioned reaction, may be isolated and purified from the reaction mixture by the known methods such as filtration, extraction, re-crystallization, chromatography etc.

Furthermore, the stereo isomers of the product may be separated by known methods such as fractional crystallization, chromatography, etc.

The pyrimidine derivatives of the present invention or the pharmaceutically acceptable salt thereof may be used as general pharmaceutical compositions in the forms of solid, semi-solid or liquid, by mixing them with general organic or inorganic carrier or excipients. These formulated compounds may be used orally, parenterally or externally. The active elements may be mixed with general carriers which are non-toxic and suitable for formulations such as tablet, pellet, capsule, poultice, suppository, solution, emulsion, suspension or any kind. There is no limit to the carriers to be used. For example, the carriers may be water, glucose, lactose, arabic gum, gelatins, etc.

The amount of administration or the effective dose of the compound of the present invention will be different depending on the age and symptom of each patient, however, generally speaking, between 0.1 and 100 mg/kg per a day will be administered to the patient for the treatment.

The administration may be divided into one to several times a day orally or parenterally, such as intravenously, intramuscularly or intraarterialy. They may also be administered in the forms of poultice, suppository or intranasal formulation.

As mentioned above, the compounds of the present invention described in the formula (I) are useful as anti-inflammatory and anti-rheumatism agents, having anti-inflammatory activity in broad sense, as well as extremely low action to cause ulcers in digestive systems. Moreover, as described in the examples below, the compounds showed remarkable efficacy in the asthma and cerebrovascular disorder models, which means they are useful for the treatment of respiratory tract disorders, such as asthma, or cerebrovascular diseases. The scavenging activity of the active oxygen species by the pyrimidine derivatives of the present invention is considered to be one of the mechanisms of the abovementioned efficacy.

The pharmacological activities of the compound of the present invention were confirmed by the following experiments.

**Carageenin induced paw edema**

The test compounds were orally administered to male SD rats, 10 per a group, which were fasted for 24 hours prior to the administration, and after one hour, 0.1 ml of 1% λ-carageenin (Picnin A, Zushi Chemical Co., Ltd.) in saline was injected subcutaneously into the right hind paw. The paw volumes were measured plethysmographically at before and 3 hours after the carageenin administration. Defining the paw volume before the injection of the λ-carageenin as the basal volume, the edema forming rates were calculated by the following equation.

$$\text{Edma Forming Rate(\%)} = \frac{\text{(Paw volume after carageenin injection)} - \text{(Paw volume before carageenin injection)}}{\text{Paw volume before carageenin injection}} \times 100$$

Compounds shown in the examples 1 -3 and 5 were tested and diclofenac was used as controls. Suppression rates of these compound are shown in Table 1.

Table 1

| Test Compounds | | |
|---|---|---|
| Number of Reference Example | Dose (mg/kg) | Suppression Rate (%) |
| 1 | 2 | 48 |
| 2 | 2 | 32 |
| 3 | 2 | 11 |
| 5 | 2 | 24 |
| Diclofenac | 10 | 51 |

## Ulcer forming activity

The test compounds were orally administered to male SD rats, 10 per a group, at the dosage of 10 mg/kg, once daily for 5 days. After the final administration, rats were fasted for 24 hours and killed by blood releasing under ether anesthesia. The stomachs were taken out and the presence of ulcers were observed.

The same experiments were done with control compounds, E-5110 [Arznein Forsch. Drug Res., 37, 930 (1987), ibid, 37, 936 (1987)] abd diclofenac.

Results are shown in Table 2 below.

Table 2

| Test Compounds (Number of Example) | 1 | 3 | 4 | 5 | 6 | E-5110 | Diclofenac |
|---|---|---|---|---|---|---|---|
| Ulcer Cases | 0/10 | 1/10 | 0/10 | 0/10 | 0/10 | 3/10 | 2/10 |

## Dilating action of bronchia by Schultz-dale reaction

The specimens were prepared by removing the bronchis of Hartle guinea pigs after sensitizing them with egg white albumin and terminating them by blood releasing under ether anesthesia. Each specimen was hanged down in the Magnus vessel filled with Tyrode's solution (saturated with gas composed of 95% oxygen and 5% carbon dioxide by bubbling at pH 7.4 and at 27°C). A tension of 1g was loaded to the specimen. After the tension became constant, histamine was added to the final concentration of 2 x $10^{-7}$ g/ml to cause the specimen to contract. The specimen was taken out from the vessel, washed thoroughly and hanged down in a different Magnus vessel filled with egg white albumin, of which final concentration was 1 x $10^{-5}$ g/ml, to cause the specimen to contract. The test compound was added by 20 μMs to the vessel untill the relaxation of the specimen was observed. As a control, theophylline in 0.1% dimethylsulphoxide aqueous solution was used. The efficacy of the test compounds was described by the suppression rate, in percentage, calculated from the contraction value obtained after the addition of the test compound, defining the contraction value caused by the egg white albumin before the addition of the test compound as 100 %.

The suppression rates of the test compounds were shown in Table 3 as $IC_{50}$ values.

The results show that compounds of the present invention have equivalent bronchodilating activity with theophylline which has been known to be highly effective against IAR. Theophylline is known to have high toxicity and its $LD_{50}$ is about 300 mixing in rat. It is obvious that the compound shown in the reference example 1 should have excellent efficacy in treatment of respiratory tract disorders such as asthma, since no abnormal symptoms were observed in SD rats when it was administered at the dose of 500 mg/kg, repeatedly for 2 weeks.

Table 3

| Test Compounds (Number of Reference Example) | IC50 (μM) |
|---|---|
| 1 | 50 |
| 2 | 67 |
| 3 | 57 |
| 4 | 43 |
| 5 | 59 |
| 6 | 44 |
| 7 | 39 |
| Theophylline | 35 |

**Brain edema**

The total left carotid artery of male mongolian gerbils were separated from surrounding tissues and exposed under the Halosen anesthesia, and when awakened from the anesthesia, the total left carotid arteries were occuled by a aneurysm forming clip. Those animals showed either one-side anesthesia, rotatory motion or intermittent cramp within 5 minutes after the occluding were selected, followed by removing the aneurysm forming clip and stitched the skin. The next day, the total left caroted artery of the selected animals, 6 per a group, were re-exposed under the Halosen anesthesia, and when these animals were awakened, the test compounds were administered intraperitoneally (2 mg/kg), followed by occluding the left total carotid artery by a aneurysm forming clip 15 minutes after the administration. After 3 hours ischemla, the clips were removed to re-perfusion. The animals were guillotined at 3 hours after the re-perfusion, immediately followed by removing out the brains, separated their left and right parts at its median line and measured each brain weights. The brains were dried in the dry heat sterilizer at 100°C for 3 days, and measured each brain weights.

Here, for the comparison, experiments with only surgical operations and stitchings were done as a operation only group, and experiments with only surgical operations, clip blockigs and stitchings and without the administration of the test compounds were done as a control group. The results (water contents) are shown in Table 4 below.

The result shows a significant brain weight increase of the occlusion side in the control group, on the other hand, no brain weight increase of the occlusion side was observed when the compound of the present invention was administered, which indicates the efficacy of the compounds in treatment of the cerebrovascular disorder.

Table 4

| Group | Occlusion Side | Normal Side |
|---|---|---|
| Operation Only | 79.92 ± 0.08 | 79.93 ± 0.10 |
| Control | 81.47 ± 1.04 | 79.89 ± 0.20 |
| Compound shown in Reference Example 7 | 79.94 ± 0.33 | 79.64 ± 0.09 |

**Scavenging activity of hydroxy radicals**

Scavenging activity of hydroxy radicals were confirmed according to the method of Kharasch et al. [J. Biol. Chem., 260, 106452 (1985)]. The method is as follows. The reactions were started by adding 80 μM iron chloride (II) to the reaction mixture containing test compounds, 30 mM sodium chloride, 1.0 mM linolenic acid, 0.08% rubrol and 100 μM hydrogen peroxide in 1% N,N-dimethylformamide aqueous solution. The time course of peroxidation reaction caused by the action of hydroxy radicals to linolenic acid were monitored by measuring the change of absorbance at 234 nm reflecting the generation of conjugate double bond, and thus the disappearance of the hydroxy radicals by test compounds was measured. The reaction suppression rates of test compounds at their concentration of 10 μM were calculated and the values are listed in Table 5 below.

The results show that the compounds of the present invention have efficacy in scavenging the hydroxy radical.

Table 5

| Test Compounds (Number of Reference Example) | Suppression Rate (%) |
|---|---|
| 1 | 59 |
| 2 | 15 |
| 4 | 18 |
| 5 | 31 |
| 6 | 25 |
| 7 | 60 |

### Example 1

**(1-methyl-3-phenyl-5-(3,5-di-tert-butyl-4-hydroxybenzylidene)- pyrimidine-2,4, 6-trione)**

3,5-di-tert-butyl-4-hydroxybenzaldehyde 1.29 g (5.5 mmol) and 1-methyl-3-phenylpyrirnidine-2,4,6-trione 1.09 g (5 mmol) were dissolved in 25 ml ethanol and refluxed for 2 hours. The reaction mixture was cooled, and the solids were filtered. Faint yellow crystal with the following physical properties was obtained (1.43g). The yield was 66 %.

Melting Point: 156 - 158 °C

Mass Spec: m/e 434 ($M^+$)

$^1$H-NMR($CDCl_3$) δ: 8.61 & 8.52 (1H, s), 8.32 & 8.26 (2H, s), 7.55 - 7.21 (5H, m), 6.08 & 6.02 (1H, s), 3.48 (3H, d), 1.47 (18H, s)

### Example 2

**(1,3-diethyl-5-(3,5-di-tert-butyl-4-hydroxybenzylidene)-pyrimidine-2,4,6-trione)**

The whole process is the same with the example 1 except for using 1,3-diethylpyrimidine-2,4,6-trione instead of 1-methyl-3-phenylpyrimidine-2,4,6-trione. The faint yellow crystal with the following physical properties was obtained with the reaction yield of 75 %.

Melting Point: 151 °C

Mass Spec: m/e 400 ($M^+$)

$^1$H-NMR($CDCl_3$) δ: 8.49 (1H, s), 8.25 (2H, s), 5.99 (1H, s), 4.03 (4H, q), 1.48 (18H,s), 1.27 (3H, t), 1.25 (3H, t)

Elementary Analysis Values: as of $C_{23}H_{32}O_4N_2$

| | C(%) | H(%) | N(%) |
|---|---|---|---|
| Calc.: | 68.97 | 8.05 | 7.00 |
| Found.: | 68.72 | 8.12 | 6.89 |

### Example 3

**(1,3-dicyclohexyl-5-(3,5-di-tert-butyl-4-hydroxybenzylidene)-pyrimidine-2,4,6-trione)**

The whole process is the same with the example 1 except for using 1,3-dicyclohexylpyrimidine-2,4,6-trione instead of 1-methyl-3-phenylpyrimidine-2,4,6-trione The faint yellow crystal with the following physical properties was obtained with the reaction yield of 71%.

Melting Point: 215 - 216 °C

Elementary Analysis Values: as of $C_{13}H_{44}O_4N_2$

| | C(%) | H(%) | N(%) |
|---|---|---|---|
| Calc.: | 73.19 | 8.72 | 5.51 |
| Found: | 73.24 | 9.07 | 5.32 |

Mass Spec: m/e 508 ($M^+$)

$^1$H-NMR (DMSO-$d_6$) δ: 8.39 (1H,s), 8.14 (2H, s), 5.93 (1H, s), 4.72 (1H, t), 1.24 - 2.42 (20H, m), 1.47 (18H, s)

**Example 4**

**(1-butyl-5-(3,5-di-tert-butyl-4-hydroxybenzylidene)-pyrimidine-2,4,6-trione)**

The whole process is the same with the example 1 except for using 1-butylpyrimidine-2,4,6-trione instead of 1-methyl-3-phenylpyrimidine-2,4,6-trione. The faint yellow crystal with the following physical properties was obtained with the reaction yield of 88%.

Melting Point: 115 - 117°C

Elementary Analysis Values: as of $C_{23}H_{32}O_4N_2$

| | C(%) | H(%) | N(%) |
|---|---|---|---|
| Calc.: | 68.97 | 8.05 | 7.00 |
| Found: | 69.17 | 8.31 | 6.86 |

Mass spec: m/e 400 ($M^+$)

$^1$H-NMR ($CDCl_3$) δ: 8.54 & 8.48 (1H,s), 8.36 &8.30 (2H, s), 6.09 & 6.06 (1H, s), 3.99 (1H, t), 1.35 - 1.65 (4H, m), 1.48 (18H, s), 0.96 & 0.95 (3H, t)

**Example 5**

**(1-cyclohexyl-5-(3,5-di-tert-butyl-4-hydroxybenzylidene)-pyrimidine-2,4,6-trione)**

The whole process is the same with the example 1 except for using 1-cyclohexylpyrimidine -2,4,6-trione instead of 1-methyl-3-phenylpyrimidine-2,4,6-trione. The faint yellow crystal with the following physical properties was obtained with the reaction yield of 96%.

Melting Point: 223 °C

Elementary Analysis Values: as of $C_{23}H_{32}O_4N_2$

| | C(%) | H(%) | N(%) |
|---|---|---|---|
| Calc.: | 70.39 | 8.03 | 6.57 |
| Found: | 70.47 | 8.13 | 6.52 |

Mass Spec: m/e 426 ($M^+$)

$^1$H-NMR (DMSO) δ: 8.34 (1H,s), 8.31 (2H, s), 4.63 (1H, t), 2.35-1.22 (10H, m), 1.49 (18H, s)

**Example 6**

**(1-phenyl-5-(3,5-di-tert-butyl-4-hydroxybeniylidene)-pyrimidine-2,4,6-trione)**

The whole process is the same with the example 1 except for using 1-phenylpyrimidine -2,4,6-trione instead of 1-methyl-3-phenylpyrimidine-2,4,6-trione. The faint yellow crystal with the following physical properties was obtained with the reaction yield of 98 %.

Melting Point: 295 - 296 °C

Elementary Analysis Values: as of $C_{25}H_{28}O_4N_2$

| | C(%) | H(%) | N(%) |
|---|---|---|---|
| Calc.: | 71.41 | 6.71 | 6.66 |
| Found: | 71.28 | 6.84 | 6.46 |

Mass Spec: m/e 420 ($M^+$)

$^1$H-NMR (DMSO-$d_6$) δ: 11.57 (1H,brs), 8.38 (1H, s), 8.33 (2H, s), 7.38 - 7.58 (5H,m), 1.48 (18H, s)

**Example 7**

**(1,3-di-p-tolyl-5-(3,5-di-tert-butyl-4-hydroxybenzylidene)-pyrimidine-2,4,6-trione)**

The whole process is the same with the example 1 except for using 1,3-di-p-tolylpyrimidine -2,4,6-trione instead of 1-methyl-3-phenylpyrimidine-2,4,6-trione. The faint yellow crystal with the following physical properties was obtained with the reaction yield of 98 %.

Melting Point: 262 - 263 °C

Elementary Analysis Values: as of $C_{33}H_{36}O_4N_2$

| | C(%) | H(%) | N(%) |
|---|---|---|---|
| Calc.: | 75.54 | 6.92 | 5.34 |
| Found: | 75.56 | 6.84 | 5.41 |

Mass Spec: m/e 524 ($M^+$)

$^1$H-NMR ($CDCl_3$) δ: 8.51 (1H,s), 8.29 (2H, s), 7.34 - 7.42 (8H, m), 2.49 (6H, s), 1.51 (18H, s)

**Example 8**

**(1-methyl-3-phenyl-5-(3,5-dimethyl-4-hydroxybenzylidene)-pyrimidine-2,4,6-trione)**

The whole process is the same with the example 1 except for using 3,5-dimethyl-4-hydroxybenzaldehyde instead of 3,5 -di-tert-butyl-4-hydroxybenzaldehyde. The faint yellow crystal with the following physical properties was obtained with the reaction yield of 93 %.

Melting Point: 203 °C

Elementary Analysis Values: as of $C_{20}H_{18}O_4N_2 \cdot 1/3H_2O$

| | C(%) | H(%) | N(%) |
|---|---|---|---|
| Calc.: | 67.41 | 5.28 | 7.86 |
| Found: | 67.59 | 5.13 | 7.92 |

Mass Spec: m/e 350 ($M^+$)

$^1$H-NMR (DMSO-$d_6$) δ: 8.34 & 8.24 (1H,s), 8.13 &8.10 (2H, s), 7.30-7.51 (5H, m), 3.31 & 3.21 (3H, s), 2.28 & 2.22 (6H, s)

**Example 9**

**(1-methyl-3-phenyl-5-(3,5-dimethoxy-4-hydroxybenzylidene)-pyrimidine-2,4,6-trione)**

The whole process is the same with the example 1 except for using 3,5-dimethoxy-4-hydroxybenzaldehyde instead of 3,5-di-tert-butyl-4-hydroxybenzaldehyde. The faint yellow crystal with the following physical properties was obtained with the reaction yield of 90 %.

Melting Point: 267 - 269 °C

Elementary Analysis Values: as of $C_{20}H_{18}O_5N_2$

| | C(%) | H(%) | N(%) |
|---|---|---|---|
| Calc.: | 62.82 | 4.75 | 7.33 |
| Found: | 62.93 | 4.80 | 7.26 |

Mass Spec: m/e 382($M^+$)

$^1$H-NMR ($CDCl_3$) δ: 8.52 & 8.40 (1H,s), 8.50 &8.01 (2H, s), 7.37 - 7.60 (5H, m), 3.93 & 3.85 (3H, s), 3.354 & 3.348 (3H, s)

**Example 10**

**(1-methyl-3-(4-fluorophenyl)-5-(3,5-di-tert-butyl-4-hydroxybenzylidene)-pyrimidine-2,4,6-trione)**

The whole process is the same with the example 1 except for using 1-methyl-3-(4-fluorophenyl)-pyrimidine-2,4,6-trione instead of 1-methyl-3-phenylpyrimidine-2 2,4,6-trione. The faint yellow crystal with the following physical properties was obtained with the reaction yield of 85%.

Mass Spec: m/e 452($M^+$)

$^1$H-NMR ($CDCl_3$) δ: 8.53 & 8.46 (1H,s), 8.25 &8.18 (2H, s), 7.20 (3H, s), 7.05 (2H,s), 6.01 & 5.98 (1H, s), 3.39 (3H, s), 1.48 & 1.42 (18H, s)

**Example 11**

**(1-methyl-3-(4-chlorophenyl)-5-(3,5-dimethyl-4-hydroxybenzylidene)-pyrimidine-2,4,6-trione)**

The whole process is the same with the example 1 except for using 1-methyl-3-(4-chlorophenyl)-pyrimidine-2,4,6-trione instead of 1-methyl-3-phenylpyrimidine-2,4,6-trione, and using 3,5-dimethyl-4-hydroxybenzaldehyde instead of 3,5-di-tert-butyl-4-hydroxybenzaldehyde. The faint yellow crystal with the following physical properties was obtained with the reaction yield of 79%.

Mass Spec: m/e 375($M^+$)

$^1$H-NMR (DMSO-$d_6$) δ: 8.25 (1H,s), 8.05 (2H, s), 7.20 - 7.70 (4H, m), 3.30 (3H, s), 2.23 (6H, s)

Example 12

The compound 10g obtained in the example 1 was mixed with lactose 35g, hydroxypropylcellulose 4.5g and magnesium stearate 0.5g, followed by compressing it into 200 tablet formulations by using known method. The tablet may be administered one per an administration for several times per a day.

## Claims

1. Pyrimidine derivatives of the following formula (I) or their pharmaceutically acceptable salt.

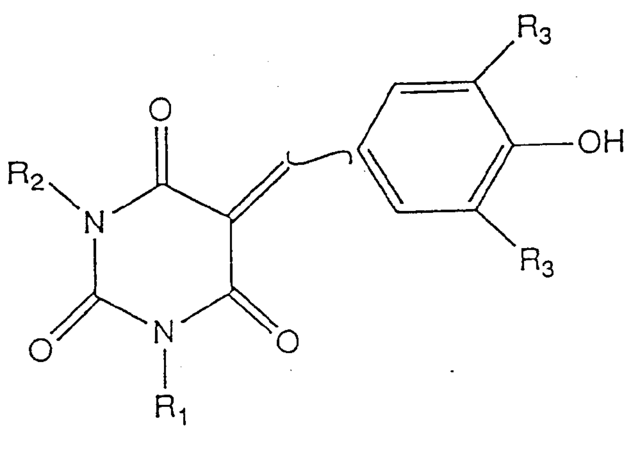

(I)

($R_1$ and $R_2$ represent independently hydrogen atom, linear or branched alkyl group, substituted or non-substituted phenyl or cyclohexyl group; and $R_3$ represents linear or branched lower alkyl or alkoxy group;

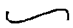 represents both stereo isomers.)

**2.** A method for preparation of the pyrimidirie derivatives of claim 1, which comprises reacting a pyrimidine derivative of the following formula (II) and a hydroxybenzaldehyde derivative. of the following formula (III).

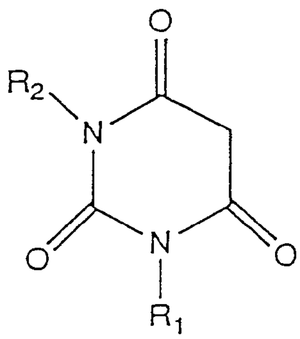

(II)

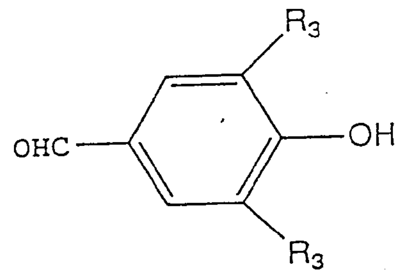

(III)

($R_1$, $R_2$ and $R_3$ represent the same as in claim 1.)

**3.** A pharmaceutical composition comprising a pyrimidine derivative or pharmaceutically acceptable salt thereof according to claim 1.

**4.** A pharmaceutical composition of claim 3 which is an anti-inflammatory agent.

**5.** A pharmaceutical composition of claim 3 which is an agent for treating respiratory tract disorders.

**6.** A pharmaceutical composition of claim 3 which is an agent for treating cerebrovascular diseases.

European Patent Office

# EUROPEAN SEARCH REPORT

Application Number

EP 92 30 9244

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 106, 1987, Columbus, Ohio, US; abstract no. 213918z, page 661 ;column 2 ; * abstract * | 1-3 | C07D239/62<br>C07D239/60 |
| X | & JP-A-6 229 570 (KANEGAFUCHI CHEM. IND.)<br>--- | 1-3 | |
| A | CHEMICAL ABSTRACTS, vol. 84, 1976, Columbus, Ohio, US; abstract no. 150652n, page 552 ;column 2 ; * abstract * | 1-3 | |
| A | & PL-A-77 134 (INSTYTUT PRZEMYSLU FARMACEUTYCZNEGO)<br>--- | 1-3 | |
| A | CHEMICAL ABSTRACTS, vol. 95, 1981, Columbus, Ohio, US; abstract no. 54821y, page 42 ;column 1 ; * abstract * & POL. J. PHARMACOL. PHARMA. vol. 33, no. 1, 1981, pages 115 - 120 TIWARI,S. ET AL. 'SEARCH FOR CNS ACTIVE AGENTS:SYNTHESIS OF N,N'BIS(POTASSIUM ALKYL/ARYL CARBOXYLATE)-5-ARYLIDENE-BARBITURIC ACIDS'<br>----- | 1-3 | |

| TECHNICAL FIELDS SEARCHED (Int. Cl.5) |
|---|
| C07D |

The present search report has been drawn up for all claims

| Place of search | Date of completion of the search | Examiner |
|---|---|---|
| THE HAGUE | 07 JANUARY 1993 | FRANCOIS J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)